# EUROPEAN PATENT APPLICATION

(11) **EP 1 415 620 A2**
(43) Date of publication of application: **06.05.2004**
(21) Application number: 03255945.2
(22) Date of filing: 23.09.2003
(51) Int. Cl.: A61F 2/36

(54) **Modular prosthetic head having a flat portion to be implanted into a constrained liner**

(30) Priority: 24.09.2002 US 413239 P; 19.09.2003 US 666168
(71) Applicant: Biomet, Inc., Warsaw, IN 46581 (US)
(72) Inventor: Gibbs, Philip Martin, Winona Lake, Indiana 46590 (US); Slone, Jason, Silver Lake, Indiana 46982 (US)
(74) Representative: Giles, Ashley Simon

(57) **Abstract**

A constraining modular hip prosthetic to reduce the possibility of a dislocation of the hip prosthetic after implantation. The constraining liner (14) is provided in a preassembled position including an opening smaller than the internal diameter of the liner. The modular head portion includes a equator diameter (28) which is small enough to be received into the liner. It includes a second dimension which is greater than the opening to constrain the modular head within the liner. A kit is also provided for trialing the modular hip prosthetic to provide and determine a customized and personal fit of the hip prosthetic for each patient.

## Description

### CROSS-REFERENCE TO RELATED APPLICATIONS

This application claims the benefit of U.S. Provisional Application No. 60/413,239, filed on 9/24/2002. The disclosure of the above application is incorporated herein by reference.

### FIELD OF THE INVENTION

The disclosure relates to joint prosthetics, and particularly to ball and socketjoint prosthetics forming a constrained joint.

### BACKGROUND OF THE INVENTION

The human body includes many mobile or articulating joints. These joints allow at least two portions of bone to move relative one another when acted upon by a force. The joints also act to keep the bones aligned, so that proper and normal functioning of the joint may occur. Nevertheless, during a lifetime different joints may become injured or affected by wear. When this occurs, the joint no longer operates as it should. The bone may easily move out of alignment or not retain its natural operating orientations. In addition, soft tissue holds the joints in place. Likewise, during a lifetime of use and wear the soft tissue may become injured or damaged, so that it no longer holds the joint as it should. When this occurs, one common effect is that the joint easily becomes dislocated. Multiple dislocations can further injure the joint and reduce the ability of the soft tissue to hold the joint in the proper orientation. At this point, a reconstruction of the joint most often is performed.

Several types of joint prosthetics are generally known in the art. A constrained prosthetic may be used when dislocation is a constant or repeated issue. The constrained prosthetic provides a ball and prosthetic socket where the ball of the prosthetic is held within the prosthetic socket or an internal cavity of the prosthetic by a mechanical means. For example, a metal ring may be placed around the opening of a liner portion disposed in the prosthetic socket to hold the ball of the joint prosthetic within the liner portion. The ring increases the lever out force needed to remove the ball from the liner portion. This makes dislocation of the ball portion from the liner portion less likely. The ring may either be assembled onto the liner portion during the manufacturing process or it may be installed during the operative procedure. Generally, however, if the ring is to be installed during the operative procedure, the liner portion must include deflectable portions separated by slits to allow the physician to install the ring.

It is also known to provide a liner that includes an internal diameter or cord that is greater than its opening. Specifically the ball portion of the joint prosthetic is keyed to the liner opening. Therefore the ball portion may be implanted in only one orientation. This allows for placement of the ball portion but resists its dislocation. The implantation orientation generally is not the natural orientation of the joint, where the ball is able to be inserted into the shell portion. Then, when the joint is moved to the natural position the ball is not easily removed from the shell. These prosthetics do not include other portions, such as a ring, which increase the lever out force of the prosthetic.

With these generally known constrained joint prosthetics, if a dislocation occurs, regardless of the constrained liner, closed reduction of the joint is generally difficult without an operative procedure. Because the constrained joint includes a portion which substantially closes the interior shell area from the ball joint, it may be difficult to perform a closed reduction of the joint into its normal orientation. Therefore, it is desirable to provide a prosthetic joint, which will include the advantages of a constrained liner, but also allow for an easy reduction of the joint if a dislocation occurs after the operative procedure.

### SUMMARY OF THE INVENTION

A joint prosthetic including a constrained shell liner to decrease the possibility of a post-operative dislocation. The constrained liner may be implanted into a prosthetic cup or into the bone itself. The ball portion of the joint being substantially spherical, but defining a cylinder around an equator, to allow implantation of the ball joint into the constrained liner. The equator defining the cylinder may be formed at any appropriate angle relative to an aperture defined by the ball, such as an aperture to receive a modular portion. The constrained liner includes an opening, into the interior or socket area. The opening or entrance has a smaller diameter or dimension than a diameter of the interior of the constrained shell. Nevertheless, the diameter of the cylinder equator of the ball is formed so that it is able to pass through the opening of the constrained liner. Generally, the cylindrical equator is placed on the ball portion of the joint, such that implantation occurs at a generally unnatural orientation of the ball portion to the shell portion of the prosthetic joint. Therefore, when the limb is placed in a natural orientation, the ball portion is substantially constrained within the constraining liner. A ring, formed of a material that is substantially rigid under normal anatomical cbnditions, such as titanium, may be placed around the opening of the constrained shell to increase the lever-out force required to dislocate the ball from the shell portion.

A first embodiment includes a prosthetic joint for replacement of a natural joint that resists dislocation. The prosthetic joint includes a liner having an internal concave portion defining a cup diameter, and defining an opening having a passage width smaller than the cup diameter. A modular ball portion has a ball diameter substantially equal to the cup diameter. A constraining ring cooperates with the opening to resist a removal of said ball portion from said liner after implantation. The ball portion includes an equator having a diameter similar to the passage width. The ball portion is adapted to be implanted into the internal concave portion during an operative procedure. The constraining ring is assembled prior to the operative procedure.

A second embodiment includes a method of implanting a joint prosthetic having a modular stem portion and modular head portion to be associated with a constraining liner after an implantation thereof. The method includes implanting a modular stem into a first boney portion and implanting a cup into a second boney portion. A trial liner is then temporarily associated with the cup. A proper modular head to operably associate the modular stem and the cup after implantation is chosen. Choosing the proper modular head includes associating a trial modular head with the modular stem, disposing the trial modular head in the trial constraining liner, and moving the first boney portion through a range of motion while the trial modular head is in the trial constraining liner. Finally, the trial portions, including trial modular heads and trial liners, are replaced by implantable portions having the appropriate range of motion, as determined by the trial portions.

A third embodiment includes a method for implanting a dislocation resistant joint prosthesis having a constraining liner affixed in a cup, and a modular head portion, extending from a modular stem member, implantable into the constraining liner. The method includes implanting the cup into a first boney portion and affixing the constraining liner to the cup. A modular stem member is implanted into a second boney portion and a modular head portion is disposed on a neck of the modular stem member. The second boney portion is oriented in an unnatural orientation and the modular head portion is inserted into the constraining liner. The second boney portion is moved to a natural orientation after the head portion is implanted into the constraining liner.

A fourth embodiment provides a kit to implant a modular hip joint. The kit includes a modular stem to be implanted into a femur and a modular head adapted to extend from the modular stem. The modular head has a major diameter. A modular trial head is adapted to cooperate with the modular stem to select the modular head. An acetabular cup is implanted into an acetabulum and a constraining liner, defining an entrance, may be affixed into the acetabular cup. A trial liner cooperates with the acetabular cup and the modular trial head during a Mating process. The entrance has a dimension less than the major diameter of the modular head. The constraining liner and the modular head interact to resist a dislocation of the modular head from the constraining liner after implantation.

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention.

### BRIEF DESCRIPTION OF THE DRAWINGS

The present invention will become more fully understood from the detailed description and the accompanying drawings, wherein:

Figure 1 is an exploded perspective view of a constrained modular hip prosthetic according to a first embodiment;

Figure 2 is a planned view of a kit for the implantation of the hip joint prosthetic;

Figure 3 is a detailed perspective view of a method for trialing the hip prosthetic;

Figure 4 is an exploded planned view of an implantation method of the hip prosthetic;

Figure 5 is a detailed partial cross-section view of the hip prosthetic in its implanted and natural position; and

Figure 6 is an exploded perspective view of a constraining modular hip prosthetic according to a second embodiment.

### DETAILED DESCRIPTION OF VARIOUS EMBODIMENTS

The following description of various embodiments is merely exemplary in nature and is in no way intended to limit the invention, its application, or uses. Although the following description specifically relates to an acetabular prosthesis, including an acetabular constrained liner and a femoral head prosthetic, it will be understood that the present invention may be used for any prosthetic joints requiring a ball portion and a socket portion. Moreover, although the following description relates to a constrained liner fixed in a prosthetic cup, the liner may also be implanted directly into a boney portion.

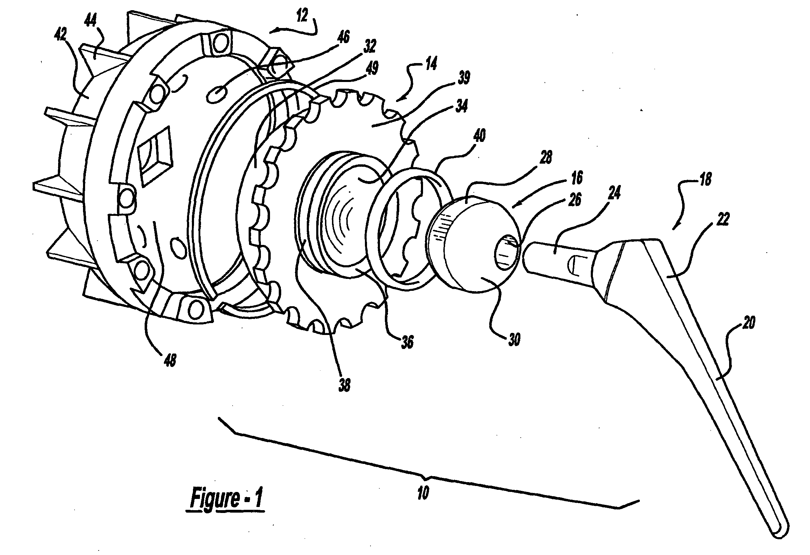

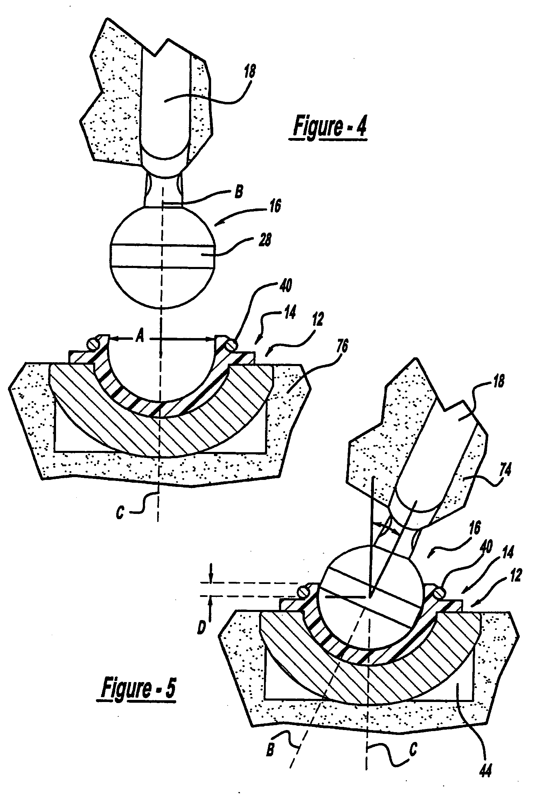

With reference to Figure 1, a joint prosthetic 10 generally includes an acetabular cup or shell 12. An acetabular liner, that is substantially solid also referred to as a constrained liner 14, a femoral head prosthetic 16, and a femoral stem prosthetic 18. Although the following discussion refers to the constrained liner fixed within an acetabular cup 12, it will be understood that the constrained liner 14 may simply be fixed into a boney structure. The femoral stem 18 generally includes a stem portion 20, which is received into the femur during implantation. A shoulder or transition portion 22 is provided between the stem 20 and a neck 24. The neck 24 generally forms a Morse taper with a female taper 26 defined in the femoral head 16. The female taper 26 and the neck 24 form a substantially friction lock junction between the femoral stem 18 and the femoral head 16. It will be understood that a taper junction may also be formed when a male taper extends from the femoral head 16 and a female taper is provided on the stem 18.

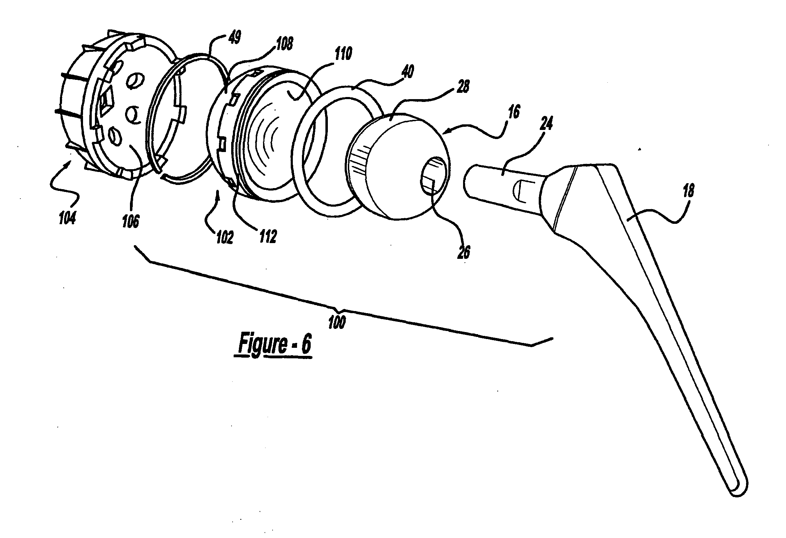

The femoral head 16 is substantially spherical. The sphere of the femoral head 16 is interrupted to allow the creation of the female taper 26 and a depressed or cylindrical equator 28. The cylindrical equator 28 is formed at an appropriate equator, as described herein, to allow for easy implantation of the femoral head 16 into the constrained liner 14, but does not allow for easy removal of the femoral head 16 from the constrained liner 14 when the modular stem 18, implanted in a femur, is in an anatomical position. The equator around which the depressed equator 28 is formed may be any equator and may be of an angle to a central axis of the femoral head 16. The cylindrical equator 28 is formed about an axis B (as shown in Figure 4) which defines a center of an implantation presentation face.

The femoral head 16 includes an exterior surface 30, which is generally highly polished to a mirror-like finish, to allow easy articulation of the femoral head 16 within the constrained liner 14. The femoral head 16 includes a major or spherical diameter which is defined as a diameter between any two points on the exterior surface 30, of the femoral head 16, and passing through the spherical center of the femoral head 16. The femoral head 16 also defines a cylindrical or equator diameter. The equatorial diameter is defined as the diameter of the cylinder defining the cylindrical equator 28. The cylindrical equator 28 may be oriented anywhere on the femoral head 16. The cylindrical equator 28 may circle the center of the femoral head 16 or may be offset therefrom. Also, the cylindrical equator 28 may be placed at any orientation relative to the female taper 26. The equatorial diameter or distance is generally smaller than the spherical or major diameter of the femoral head 16.

The constrained liner 14 includes an exterior hemisphere 32 and an interior hemisphere or socket portion 34. On a distal side of the liner 14 is defined a liner projection 36. It will be understood that the exterior and the interior of the constrained liner 14 may not be exactly a hemisphere. Extending between the projection 36 and the exterior of the liner 14 is a land area 39. The liner projection 36 is substantially annular and includes an annular track 38, defined on the exterior thereof. The annular track 38 receives a constraining ring 40 during production. It will be understood that the constraining ring 40 may also be installed in the annular track 38 intraoperatively. The constraining ring 40 may be placed into the annular track 38 using any appropriate means. For example, the ring 40 may be mechanically forced over the projection 36 and into the annular track 38. Nevertheless, the ring 40 is generally placed in the track 38 pre-operatively.

The acetabular cup 12 defines an exterior 42, which is placed in a prepared acetabulum. Extending from the exterior of the acetabular cup 12 are spikes or projections 44 that may be used to imbed the acetabular cup 12 into the acetabulum. In addition, a plurality of screw holes 46 are provided to allow screws to be placed through the acetabular cup 12 to be received in the acetabulum. It will be understood that only one or neither of the spikes 44 or the screw holes 46 may be provided, moreover the cup 42 may be fixed with a cement. The interior of the acetabular cup 12 defines a concave hemisphere portion 48. The diameter of the internal hemisphere 48 of the acetabular cup 12 is substantially equal to an exterior diameter of the exterior convex surface of the constrained liner 14. Therefore, the acetabular liner 14 is substantially received into the acetabular cup 12. If the constrained liner 14 is, however, not hemispherical, then the interior of the cup 12 will also not be a hemisphere. Any appropriate means may be used to secure the acetabular liner 14 into the acetabular cup 12. For example, a locking ring 49 or a bone cement may be used to fix the acetabular liner 14 into the acetabular cup 12. It will also be understood that an appropriate constrained liner 14 may not need to be implanted in the cup 12, but rather implanted directly into the acetabulum.

The appropriate size of the acetabular shell 12 may be determined either pre- or intra-operatively. Varying sizes of the acetabular cup 12 and the acetabular liner 14 may be provided to customize a fit for a particular patient. Nevertheless, a single size of the femoral head 16 may be provided because of the modular acetabular and femoral implant. Therefore, the interior hemisphere 34 of the acetabular liner 14 remains the same and similar to the exterior diameter of the femoral head 16. Nevertheless, it will be understood that a plurality of femoral head diameters may be provided which would be implanted with related sized constrained liners. Having a single size femoral head 16 can allow a reduction in inventory only requiring varying taper depths.

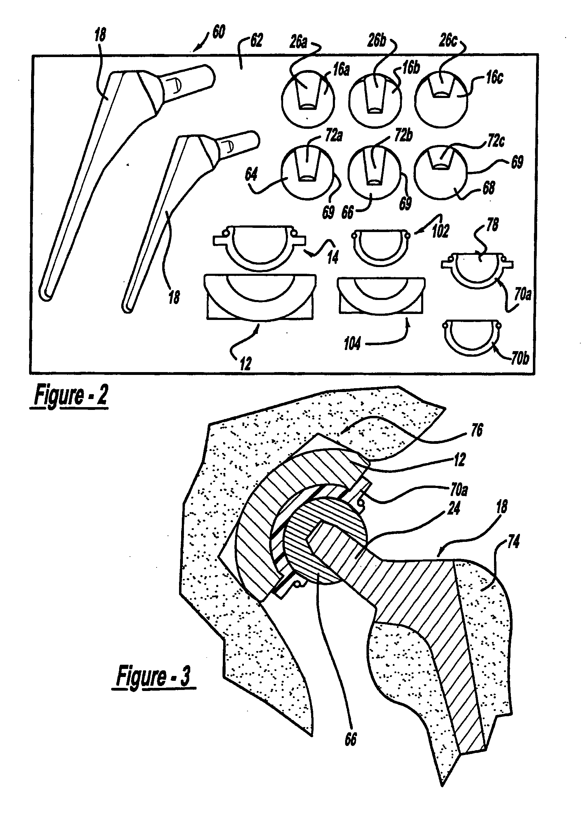

With reference to Figure 2, a kit 60 allows for a substantial personalization or custom fit of the prosthetic 10 to the particular patient. As described herein, a trial and implantation method may be used in conjunction with the kit 60 to assure a most preferred fit of the joint prosthetic 10. This customization allows for a more precise and natural orientation of the femur to the pelvis. The kit 60 may include a container 62, which includes multiple femoral stems 18, the multiple acetabular liners 14, 102 and the multiple acetabular cups 12, 104. Also included in the kit 60 are a plurality or set of femoral trial heads 64, 66 and 68 each having a cylindrical diameter 69. It will be understood that although the set of femoral trial heads includes three any appropriate number of trial heads 64, 66, 68 may be included. Also included is a set of trial liners 70a, 70b, that correspond to one of the implant constraining liners 14, 102. Finally, a set of final or implantable femoral heads 16a, 16b, and 16c are provided in the kit 60. Although the kit 60 is here illustrated to include both the trial and implant portions, this is not intended to be a limiting example. For example, the kit 60 may only include the trial portions while the implant portions are kept separately, and only opened when the appropriate size has been selected. In this instance, the kit 60 would substantially be a trial kit and only include the trial heads 64, 66, 68 and the trial liners 70a and 70b.

Each of the implantable heads 16a, 16b, 16c includes a feature substantially equivalent to one of the trial femoral heads 64, 66, 68. Generally, the trial heads 64, 66, 68 have a trial female taper 72 that has an associated and varying depth. Each of the implantable femoral heads 16a, 16b, 16c includes a depth of the female taper 26a, 26b, 26c which is equal to one of the female tapers 72 of the trial heads 64, 66, 68. Therefore, the kit 60 allows for a trialing of the joint prosthetic 10 to insure a proper and customized fit. The varying depth of the female tapers 26a, 26b, 26c effectively varies the length of the neck 24, which varies the distance between the stem 18 and the constrained liner 14. The shallower the female taper 26 the further the stem 18 is held from the liner 14. This both aligns the femur and properly tensions the soft tissue.

With reference to Figures 2-5, one method for trialing and implanting the prosthetic joint 10, including the kit 60, includes implanting the femoral stem 18 into a femur 74. The femur 74 may be prepared to receive the femoral stem 18 using any appropriate and generally known method. Likewise, an acetabulum 76 is prepared to receive the acetabular cup 12. Methods of preparing the acetabulum 76 are also generally known in the art and any are appropriate to prepare the acetabulum 76 for the acetabular cup 12. After the femur 74 and the acetabulum 76 are prepared, the femoral stem 18 is implanted into the femur 74 and the acetabular cup 12 is implanted into the acetabulum 76. As discussed above, an appropriate liner may be provided which is implanted directly into the acetabulum 76.

During the trialing phase of the operative procedure, the trial liner 70 is temporarily placed into the acetabular cup 12. Although the trial liner 70 is illustrated to include a constraining ring in a constraining format, and may be so formed in the trial procedure, it is not necessary that the trial liner 70 be a constraining liner. Rather, the trial liner 70 may simply include a shape or size that substantially mimics the shape and size of the constraining implant liner. This may increase the ease of the trial process by not constraining the trial head within the trial liner and thus decreasing the time required for the trialing procedure. Therefore, it will be understood that the trialing liner 70 may either include a constraining portion or not include a constraining portion. The trial liner 70 may be held in place with a temporary cement or other appropriate temporary methods. For example, the ring lock 49 may be used to temporarily hold the trial liner 70 in place. The trial liner 70 has an opening or entrance 78 similar or the same as the opening in the acetabular liner 14. The cylindrical diameter 69 of the trial heads 64, 66, 68 is small enough to allow easy placement in and removal from the trial liner 70 while simulating the prosthetic. This allows for easy trialing of the various femoral trial heads 64, 66, 68 and for speed and efficiency of the trialing procedure. A trial constraining ring may also be included on the trial liner 70, to further simulate the constraining liner 14. The trial liner 70 is designed to simulate the constraining liner 14 for range of motion and positioning of the femur after implantation.

The physician may choose the first trial femoral head 64, which includes the female taper having a depth of about 3mm. The physician may place the trial femoral head 64 onto the neck 24 of the femoral stem 18. The physician may then place the femoral stem 18, including the trial head 64 into the trial liner 70. The physician then determines whether an appropriate fit has been obtained. If not, the physician may then attempt to trial the second femoral head 66, including a female taper 72b, having a depth of approximately 2mm. Again, the physician may test a range of motion and orientation using the second femoral trial head 66 by placing it into the trial liner 70.

The physician may trial each of the trial femoral heads 64, 66, 68 to determine the appropriate length of the female taper 26. The physician may also trial each of the trial liners 70a and 70b and trial acetabular cups. The trial liners 70a and 70b are placed in the acetabulum 76 or the cup 12 and the femoral head is placed therein and moved through a range of motion to check for early impingement. If early impingement is discovered, the physician may move the trial liner 70a, 70b or choose a different trial liner. In any case, the physician can trial for both liner impingement and proper soft tissue tightness with the trial liners 70a, 70b, and the trial heads 64, 66, 68. Therefore, a complete customization of the prosthetic implant 10 can be obtained. Although not illustrated, the liner 14, 102 need not include only a flat face liner. It may include an internal slant or a high wall. When the prosthetic liner to be implanted is not a flat face liner, such as one that includes a highwall or internal degree, the physician may also trial the position and orientation of the trial liner 70a, 70b. Nevertheless, due to the orientation and shape of the trial acetabular femoral heads 64, 66, 68 a trial can be performed before implanting the constrained liner 14 in its final orientation. Once the physician has determined the appropriate length of the female taper 26, the physician then chooses the appropriate femoral head 16a, 16b, or 16c. The femoral heads 16 include the female taper 26 of equivalent depth to one of the trial femoral heads 64, 66, 68. Therefore, an appropriate femoral head 16 cup 12 position and liner 14 position, may be chosen through the trialing procedure and the kit 60.

With reference to Figures 4 and 5, a method of implanting and reducing the joint prosthetic 10 is illustrated. Once the appropriate femoral head 16 has been chosen, it is placed on the neck 24 of the femoral stem 18, while the joint is dislocated. Also, the constrained liner 14 is affixed in the selected orientation into the acetabular cup 12. The constrained liner 14 already has installed the constraining ring 40 so that once the femoral head 16 is implanted into the constrained liner 14 the femoral head 16 cannot be easily removed therefrom. Moreover, because the constraining ring 40 is already installed on the constrained liner 14 the physician need not install the constraining ring 40 during the operative procedure. The cylindrical equator 28 of the femoral head 16 allows it to be implanted into the acetabular liner 14 with the constraining ring 40 in place. Nevertheless, as discussed above, the constraining ring 40 may be provided separately from the constrained liner 14 and the constrained ring 40 may be implanted intra-operatively by the physician.

The projection 36 of the acetabular liner 14 defines an opening or entrance diameter A (illustrated in Figure 4). The opening diameter A is similar to the diameter of the cylindrical equator 28. The opening diameter A may be equal to, slightly smaller or larger than the diameter of the cylindrical equator 28. For example, if the diameter A has length of about 34mm, the diameter of the cylindrical equator 28 may be about 34.2mm. Therefore, with an acceptable amount of force the femoral head 16 may be pushed through the entrance defined by the projection 36 and into the concave interior 34 of the acetabular liner 14. Nevertheless, the distance A may be any appropriate length. This is done by aligning an axis B of the cylindrical equator 28 substantially coaxial with a central axis C of the acetabular liner 14. Although an axis B is illustrated to be generally aligned with the stem 18 it does not necessarily need to be so. For example, the axis B of the cylindrical portion 28 may be formed at an angle relative to the stem 18. Therefore, it will be understood that the axis B defined by the cylindrical portion 28 may be formed at any angle relative to the stem portion 18 or a portion of the head, such as the aperture 26 to receive a portion of the stem 18, at any appropriate angle and may be about 0 to about 180° relative thereto. When this occurs, an implantation face of the femoral head 16 is presented and is substantially equal to the diameter A of the opening of the liner 14. Therefore, the femoral head 16 may be received into the concave interior 34 of the acetabular liner 14. Generally, after the femoral head 16 has been implanted onto the neck 24 of the femoral stem 18, the axis B of the cylindrical equator 28 is coaxial with the axis C of the acetabular liner 14 when the femoral stem 18 and the femur are in an unnatural or non-anatomical position. Therefore, it is less likely that this orientation will occur after implantation is completed.

With reference to Figure 5, once the femoral head 16 is implanted into the acetabular liner 14 and placed in an anatomical position, the axis B of the cylindrical equator 28 is no longer coaxial with the axis C of the acetabular liner 14. Essentially, the axis B and the axis C intersect when the stem 18 is placed in a natural or anatomical position. Therefore, because the diameter of the femoral head 16 is greater than the diameter A of the acetabular liner 14, the femoral head 16 may not easily dislocate from the acetabular liner 14 when the axes B and C intersect. In addition, the constraining ring 40 assists in making rigid and stronger the projection 36 surrounding the opening of the acetabular liner 14. Therefore, the rigidity of the acetabular liner 14, generally formed of a high molecular weight polyethylene, is reinforced by the constraining ring 40. Moreover, post operatively, the acetabular liner 14 extends a distance D above a hemisphere of the femoral head 16. The distance D is generally less than about 15 mm. This is necessary to provide a force, which will constrain the femoral head 16 within the acetabular liner 14 after implantation.

With reference to Figure 6, a prosthetic joint 100 according to a second embodiment is illustrated. The prosthetic joint 100 includes the femoral stem 18 and the femoral head 16. The femoral head 16 again includes a female taper 26 and a cylindrical equator 28. Moreover, the prosthetic joint 100 includes the constraining ring 40, which is received on an acetabular liner 102. The acetabular liner 102 is received in an acetabular cup 104. The acetabular cup 104 includes an exterior, which has a diameter, which is smaller than the exterior diameter of the acetabular cup 12. Therefore, the acetabular cup 104 also includes a concave interior 106, which has a diameter smaller than the diameter of the concave interior of the acetabular cup 12. This, in turn, allows that the exterior diameter of the convex portion of the acetabular liner 102, to be smaller.

Due to this smaller size, the acetabular liner 102 does not include a land area similar to the land 39 of the acetabular liner 14. Nevertheless, the concave interior 110 of the acetabular liner 102 can be substantially similar in size to the interior of the acetabular liner 14. Therefore, the single femoral head 16 may be used in any size acetabular cup 12, 104. In addition, a single constraining ring 40 can be received on a plurality of sizes of acetabular liners 14, 102. The acetabular liner 102 includes an annular track 112, which receives the constraining ring 40. According to this embodiment, the thickness of the walls of the acetabular liner 102 may be thinner than the walls of the larger acetabular liner 14, but are still appropriate for the implantation into the acetabular cup 104.

The cylindrical equator 28 allows the femoral head 16 to be inserted into the constraining liner 14, 102 regardless of the size of the femoral head 16. The femoral head 16, therefore, can be large enough to provide an optimum range of motion once implanted into the patient. Moreover, the constraining liner 14, 102 includes a high lever out strength due to the fact that the projection 36 is substantially solid and does not include any breaks or slots therein. Therefore, when a lever out force is applied through the femoral head 16, the force is distributed through a substantial portion or all of the projection 36 and the ring 40. Again, this allows for the use of larger diameters for the femoral head 16 without increasing the possibility of a dislocation due to a lever out of the femoral head 16 from the constraining liner 14, 102.

In conjunction with the substantially spherical femoral head 16, the distance D that the acetabular liner 14, 102 extends beyond the hemisphere of the femoral head 16 also allows a great range of motion. Rather than extending a longer distance above the hemisphere of the femoral heads 16, the shape of the femoral head 16 substantially holds the femoral head 16 in the acetabular liner 14, 102. The shape of the femoral heads 16 cooperate with the distance D to ensure that the femoral head 16 does not easily dislocate from the acetabular liner 14, 102. Essentially, the diameter of this sphere defined by the femoral head 16 is substantially equal to the diameter of the hemisphere defined by the acetabular liner 14, 102. Moreover, the diameter of the femoral head 16 is larger than the opening A of the acetabular liner 14, 102. It is only the inclusion of the cylindrical equator that allows for an implantation of the femoral head 16 into the acetabular liner 14, 102. The interaction of these features allows a range of motion, after implantation, of at least about 80°. Nevertheless, the distance D can be increased and still allow for a great range of motion because of the sizes of the femoral head 16 allowed due to the inclusion of the cylindrical equator 28. Therefore, a distance D that increases the lever out strength of the prosthetic 10, 100 will still allow a large range of motion.

One measure of lever out force is the force generally created when the neck 24 of the stem 18 engages the projection 36 of the acetabular liner 14 and creates a force that attempts to dislocate the femoral head 16 from the internal cavity 34. This force is resisted by the interaction of the surface 30 of the femoral head 16 with the projection 36 which is reinforced by the constraining ring 40. It will be understood that other definitions of lever-out force or strength are generally known and may also be increased through use of the constrained liner 14, 102 and the femoral head 16.

Therefore, the joint prosthetic 10, 100 can be provided that includes a constrained liner to reduce the possibility of a dislocation postoperatively of the prosthetic joints 10, 100, which does not include the physician being required to do any more than implant the proper size of the acetabular cup 12, 104 and femoral head prosthetic 16. In addition, the implantation of the femoral head prosthetic 16 into the acetabular liner 14, 102 only requires the proper orientation of the femoral head 16. Therefore, excessive force is not required to implant the femoral head 16 into the acetabular liner 14, 102. Moreover, because the constraining ring is provided before the operative procedure begins, the steps required to implant the prosthetic joint, 10, 100 are reduced. Nevertheless, the advantages included in having the constraining ring 40 provided with the joint prosthetic 10, 100 are retained.

Moreover, if a post operative dislocation occurs, of the prosthetic joint 10, 100 a closed reduction of the prosthetic joint 10, 100 can occur without need for additional open surgery. This can be done by moving the femoral head 16 to the proper orientation, such that the axis B of the prosthetic femoral head 16 is aligned with the axis C of the acetabular liner 14, 102.

The cylindrical equator defined by the head portion that is generally held within the constraining liner may operably interact with the liner such that a non-sealing fit is formed with a portion of the liner during physical use. This allows a fluid, such as a natural or biological fluid, to flow between or through the head portion and the liner portion. The fluid may reduce friction between the head or ball portion and the liner portion after implantation. In addition, the presence of the fluid may increase lever out or pull out forces while reducing wear on the liner portion. This may increase the longevity of the implant and decrease the necessity for revision procedures. In addition, because of the cylindrical equator, the entire surface area of the sphere which is generally defined by the head portion or ball portion, does not contact the liner. That is because of the cylindrical equator or a portion of the entire sphere or arc of the head does not contact the liner at a given time. This may also reduce wear on the liner over a plurality of cycles after implantation of the implant. This may again increase longevity and reduce the possibility of a revision procedure due to wear on the liner.

It will also be understood that the head portion need not necessarily be used with a constraining liner. The head, with the cylindrical equator, may be used with any appropriate liner or acetabular implant. As discussed above the cylindrical equator may reduce wear and reduce the need for a revision procedure. Though this theory is not intended to limit the scope of the appended claims, simply that the head need not only be used in a constrained liner.

The description of the invention is merely exemplary in nature and, thus, variations that do not depart from the gist of the invention are intended to be within the scope of the invention. Such variations are not to be regarded as a departure from the spirit and scope of the invention.

## Claims

1. A prosthetic joint for replacement of a natural joint to resist dislocation, the prosthetic joint comprising:
a liner including an internal concave portion defining an internal concave diameter, and defining an opening having a passage width smaller than said internal concave diameter;
a ball portion having a ball diameter substantially equal to said internal concave diameter; and
a constraining ring cooperating with said opening;
wherein said ball portion includes an equator having a diameter similar to said passage width;
wherein said ball portion is adapted to be implanted into said internal concave portion during an operative procedure.

2. The prosthetic joint of claim 1, further comprising:
a cup adapted to be implanted into a first boney structure; and
a stem adapted to be implanted into a second boney structure;
wherein said ball portion is a head and is adapted to extend from said stem;
wherein said liner is affixed to said cup such that said ball portion is able to articulate within said internal concave portion.

3. The prosthetic joint of claim 2, wherein said ball portion further defines a cylindrical equator having a cylinder diameter to allow said ball portion to be inserted into said internal concave portion through the opening.

4. The prosthetic joint of claim 3, wherein said cylinder diameter is alignable with said opening in only one insertable orientation, said insertable orientation includes an unnatural orientation of said second boney portion relative to said first boney portion.

5. The prosthetic joint of claim 4, wherein said insertable orientation substantially aligns an axis of said cylindrical equator and said liner.

6. The prosthetic joint of claim 2, wherein said ball portion and said stem are substantially modular and operably interconnect for use.

7. The prosthetic of claim 3, wherein said ball portion generally defines a sphere including an equator and a sphere diameter, wherein said cylinder diameter is formed at said equator such that having a diameter smaller than said sphere diameter.

8. The prosthetic joint of claim 2, wherein said ball portion is resistant to dislocation from said internal concave portion through interaction of said ball portion with said opening of said liner.

9. The prosthetic joint of claim 3, wherein said cylindrical equator is disposed at an angle relative to said stem.

10. The prosthetic joint of claim 1, wherein:
said constraining ring is assembled onto said liner prior to an operative procedure to implant said liner; and
said constraining ring resists a removal of said ball portion from said liner after implantation.

11. The prosthetic joint of claim 1, further comprising:
said liner is adapted to be fixed directly to a first boney structure; and
a modular stem adapted to be implanted into a second boney structure;
wherein said ball portion is a modular head and is adapted to extend from said modular stem;
wherein said ball portion is able to articulate within said internal concave portion.

12. The prosthetic joint of claim 1, wherein:
said constraining ring is assembled onto said liner during an operative procedure to implant said liner; and
said constraining ring resists a removal of said ball portion from said liner after implantation.

13. The prosthetic joint of claim 3, wherein said cylindrical diameter is operable to allow a flow of a fluid through said cup.

14. A method of implanting a joint prosthetic having a modular stem portion and a modular head portion to be associated with a constraining liner after an implantation thereof, the method comprising:
implanting a modular stem into a first boney portion;
implanting the constraining liner to operably associate with a second boney portion;
determining a proper modular head to operably associate said modular stem and said constraining liner after implantation, including:
associating a trial modular head with said modular stem;
disposing said trial modular head in the constraining liner;
moving said first boney portion through a range of motion while said trial modular head is in said constraining liner; and
replacing said trial modular head by implanting a modular head that is associated with said trial modular head having the appropriate range of motion.

15. The method of claim 14, wherein implanting the constraining liner to operably associate with a second boney portion includes:
temporarily associating a trial liner with said second boney portion; and
performing the determination of a proper modular head to operably associate said modular stem and said constraining liner after implantation by trialing the trial femoral head in said trial liner;
wherein said trial liner has a size or shape substantially similar to said constraining liner to substantially simulate a range of motion of said modular head when in said constraining liner.

16. The method of claim 15, wherein temporarily associating a trial constraining liner with said cup includes:
disposing a trial liner including a trial entrance having a dimension similar to said liner entrance dimension; and
fixing said trial liner in a selected orientation relative a cup to allow said modular head to be disposed and moved within said trial liner.

17. The method of claim 16, wherein determining a modular head to be implanted with said modular stem further includes:
selecting a trial modular head having a first taper length;
operatively interconnecting said modular stem and said trial liner with said selected trial modular head; and
determining a proper fit of said modular stem with said selected trial modular head;
wherein a proper fit allows for a substantially natural orientation of said first boney structure relative to said second boney structure with substantially no impingement;
wherein a plurality of said trial modular heads are tested to determine said proper fit;
wherein said modular head is selected to be implanted with a taper length equivalent to said first taper length when a proper fit is found.

18. The method of claim 14, wherein implanting the constraining liner to operably associate with a second boney portion includes:
implanting a cup in a second boney portion; and
implanting the constraining liner to operably associate with said cup;
wherein said constraining liner extends from said cup which is implanted into said second boney portion.

19. The method of claim 14, further comprising:
said constraining liner having an entrance including a liner entrance dimension;
implanting said determined modular head into said constraining liner, including:
aligning a cylindrical equator of said modular head with said entrance;
displacing said modular head through said entrance; and
orienting said modular stem to a natural position so that said cylindrical equator is substantially not aligned with said entrance.

20. The method of claim 14, further comprising:
said constraining liner defining a central axis;
aligning an axis of a cylindrical equator of said modular head with said central axis;
displacing said modular head into said constraining liner; and
positioning said modular head such that said axis of said cylindrical equator intersects said central axis.

21. A method for implanting a dislocation resistant joint prosthesis having a constraining liner and a modular head portion, extending from a modular stem member, implantable into the constraining liner, the method comprising:
affixing the constraining liner such that said constraining liner operably extends from a first boney portion;
implanting the modular stem member into a second boney portion;
disposing the modular head portion on a neck of said modular stem member;
orienting said second boney portion in an unnatural orientation;
inserting the head portion, while the second boney portion is in the unnatural position, into the constraining liner; and
moving the second boney portion to a natural orientation after the head portion is implanted into the constraining liner.

22. The method of claim 21, further comprising:
trialing a plurality of trial modular heads to determine a proper modular head including:
temporarily associating a trial constraining liner with said cup;
disposing a trial liner including a trial entrance having a dimension similar to said liner entrance dimension;
fixing said trial liner in a selected orientation relative said cup to allow said trial modular head to be disposed and moved within the trial liner; and
measuring an orientation of said first boney structure relative to said second boney structure.

23. The method of claim 22, further comprising:
moving said second boney portion through a range of motion relative to said trial liner to determine an early impingement; and
adjusting said trial liner when an early impingement occurs to reduce or eliminate the early impingement.

24. The method of claim 22, wherein trialing a plurality of trial modular heads further includes:
selecting a trial modular head having a first taper length;
operatively interconnecting said modular stem and said trial liner with said selected trial modular head; and
determining a proper fit of said modular stem with said selected trial modular head;
wherein a proper fit provides said first boney portion and said second boney portion is a substantially natural orientation;
wherein said modular head is selected to be implanted with a taper length equivalent to said first taper length when a proper fit is found.

25. The method of 21, further comprising:
implanting the cup into the first boney portion; and
disposing the constraining liner in the cup:
wherein said liner is implanted to receive the head portion.

26. The method of claim 21, wherein said head portion includes a cylindrical equator and orienting said second boney portion in an unnatural orientation includes:
disposing the modular head portion in an orientation such that an implantable cross section is presented to an entrance of the constraining liner such that the head portion is implantable into the constraining liner; and
inserting the modular head portion includes displacing the modular head portion into the constraining liner while the implantable cross section remains presented to the entrance.

27. The method of claim 21,
wherein the constraining liner has an entrance including a liner entrance dimension;
implanting said determined modular head into said constraining liner, including:
aligning a cylindrical equator of said modular head with said entrance;
forcing said modular head through said entrance; and
orienting said modular stem to a natural position so that said cylindrical equator is substantially not aligned with said entrance.

28. The method of claim 21, wherein said head portion includes a cylindrical equator defining an axis and said constraining liner includes a central axis and orienting said second boney portion in said unnatural position includes:
causing said axis of said cylindrical equator to substantially align with said central axis.

29. A trial kit to trial for the implantation of a modular hip joint having a modular femoral head and a constraining liner, comprising:
a modular trial head having an implantation face operable to cooperate with a modular stem and assist in the selection of a modular head;
a trial liner having an entrance dimension and adapted to cooperate with a boney structure and said modular trial head during a trialing process;
wherein said entrance dimension is operable to cooperate with said modular trial head to substantially mimic the constraining liner;
wherein said constraining liner and said modular head interact to resist a dislocation of said modular head from said constraining liner after implantation.

30. The trial kit of claim 29, further comprising:
a modular stem to be implanted into a femur;
a modular head adapted to extend from said modular stem, having an implantation face;
an acetabular cup to be implanted into an acetabulum;
a constraining liner, to be affixed into said acetabular cup, defining an entrance.

31. The trial kit of claim 30, further comprising:
a plurality of said modular heads, wherein each modular head defines a female or male taper each having a different taper length;
a plurality of said modular trial heads, wherein each modular trial head defines a female or male taper length equivalent to one of the different lengths of said female or male taper of said modular head;
wherein a major diameter of each modular trial head is smaller than said major diameter of said modular head;
wherein said plurality of modular trial heads are used to select one of said plurality of modular heads for implantation.

32. The trial kit of claim 31, wherein each of said taper lengths provides a different orientation of said modular stem to said constraining liner.

33. The trial kit of claim 30, further comprising:
a constraining ring associated with said entrance of said constraining liner to substantially increase a rigidity of said entrance.

34. The trial kit of claim 30, wherein said modular head defines a cylindrical equator having an equator diameter adapted to allow said modular head to pass through said entrance; wherein when said modular head is implanted in said constraining liner said major diameter is aligned with said entrance.

35. A prosthetic joint for replacement of a natural joint, the prosthetic joint comprising:
a first member including an internal concave portion defining an internal concave diameter;
a second member having a selected diameter and defining at least one cylindrical portion about at least a portion of a selected equator of said second member;
wherein said ball portion is adapted to be implanted into said internal concave portion during an operative procedure;
wherein said cylindrical portion is operable to substantially ensure contact with less than the entire internal concave portion.

36. The prosthetic joint of claim 35, wherein said first member includes a liner operable to resist removal of said second member from said internal concave portion after implantation therein.

37. The prosthetic joint of claim 35, wherein said second member defines at least a portion of a sphere and said cylindrical portion is positioned at a selected equator of said sphere and includes a radius less than a radius of said sphere to a point outside of said cylindrical portion.

38. The prosthetic joint of claim 35, wherein said cylindrical portion is operable to allow a selected fluid to flow into said internal concave portion after said second member is positioned in said first member.

39. A prosthetic for replacement of an anatomical portion, comprising:
a first member including an internal concave portion defining an internal concave diameter; and
a second member having a first diameter substantially equal to said internal concave diameter and defining at least a portion of an equator having a second diameter less than said first diameter;
wherein said second member is adapted to be implanted into said internal concave portion during an operative procedure;
wherein said equator substantially eliminates complete contact of said second member with said first member.

40. The prosthetic of claim 39, wherein said second member substantially defines a sphere and said equator is an equator of said sphere generally defining a cylindrical portion including a selected height.

41. The prosthetic of claim 40, wherein said first member and said second member articulate relative to each other;
wherein said equator defined by said second member is operable to reduce wear by contacting said internal concave portions on a surface area less than a surface area of a surface of the sphere.

42. The prosthetic of claim 39, wherein said equator defined by said second member allows a passage into said internal concave portion of said first member after said second member is implanted relative to said internal concave portion;
wherein a selected fluid may flow into said internal concave portion through said passage.

43. The prosthetic of claim 39, wherein a passage width of said first member is substantially equivalent to said second diameter of said second member;
wherein said first diameter of said second member is greater than said passage width of said first member;
wherein said second member is operable to be implanted into said first member of a first orientation and constrained within said first member in a second orientation.

44. The prosthetic of claim 40, wherein said second member defines a transition from said cylindrical portion to reduce a wear on said first member.
